# EUROPÄISCHE PATENTANMELDUNG

(11) **EP 0 704 193 A1**
(43) Veröffentlichungstag der Anmeldung: **03.04.1996**
(21) Anmeldenummer: 95114676.0
(22) Anmeldetag: 18.09.1995
(51) Int. Cl.: A61F 2/30

(54) **Verfahren zur Herstellung einer Endoprothese als Gelenkersatz bei Kniegelenken**

(30) Priorität: 27.09.1994 DE 4434539
(71) Anmelder: Schuster, Luis, Dr.med., D-86911 Riederau (DE)
(72) Erfinder: Schuster, Luis, Dr., D-86911 Riederau (DE); Schuster, Christoph, D-82031 Grünwald (DE)
(74) Vertreter: Gauger, Hans-Peter, Dipl.-Ing.

(57) **Zusammenfassung**

Bei einem Verfahren zur Herstellung einer Endoprothese als Gelenkersatz insbesondere bei Kniegelenken bildet ein Vergleichsbild zur Ermittlung von Konturenunterschieden an dem Femur und der Tibia, die durch den Vergleich eines korrigierten präoperativen Bildes von dem geschädigten Kniegelenk mit einem postoperativen Bild erhalten werden, die Basis für die Herstellung von entsprechenden femoralen und tibialen Komponenten der Endoprothese.

## Beschreibung

Die Erfindung bezieht sich auf ein Verfahren zur Herstellung einer Endoprothese als Gelenkersatz bei Kniegelenken, wobei die Prothese einen operativen Eingriff am Femur, der Tibia und der Patella eines geschädigten Kniegelenks voraussetzt.

Der operative Eingriff an einem Kniegelenk wird von dem behandelnden Arzt dann in Betracht gezogen, wenn der Patient über schwere Knieschmerzen und Behinderungen als Folge bspw. einer reumathischen Arthritis oder auch sonstiger Gelenkkrankheiten klagt. Der operative Eingriff verläuft dabei herkömmlich in einer Vielzahl von Stufen, die dabei ausschließlich angepaßt werden an die Formgebung von industriell in verschiedenen abgestuften Größen zur Verfügung gestellten Gelenkformteilen, die letztlich eine solche Befestigung an gefrästen Flächen hauptsächlich des vorderen femoralen Condylus, des distalen Femur, der proximalen Tibia und der Patella erfahren, daß mit solchen Komponenten eine senkrechte Ausrichtung zu einer Achse erhalten wird, die bspw. mittels einer voroperativen Röntgenfilmaufnahme und eines Marknagel-Ausrichtungssystems für die gerade Verbindungslinie zwischen der Hüftmitte, dem Knie und dem Fußknöchel erhalten wird. Eine illustrative Darstellung eines solchen operativen Vorgehens findet sich bspw. in dem Firmenprospekt 97-5110-102 20 MA der Firma Zimmer, Inc., Ausgabe 1989 "MG II TOTAL KNEE SYSTEM SURGICAL TECHNIQUE" sowie in der US-PS 4 759 350, in welcher ein betreffendes Marknagelsystem beschrieben wird.

Die Implantantion solcher dreiteiliger Kniegelenk-Endoprothesen ist nicht nur sehr aufwendig, vielmehr wird damit unter Berücksichtigung des häufig doch recht unterschiedlichen Wuchses der Patienten auch allenfalls nur eine angenäherte Wiederherstellung der Verhältnisse eines gesunden Kniegelenks erhalten. Häufig treten daher auch Komplikationen auf, die hauptsächlich auf die Mechanik der implantierten Prothesekomponenten zurückzuführen sind und so bspw. ein vorderes Kniegelenk-Schmerzsyndrom ergeben, welches durch ein Fehlgleiten der Kniescheibe mit einer unphysiologischen Belastung des Oberschenkel-Kniescheibengleitgelenks verursacht wird. Auch ergeben sich häufig Reizzustände gelegentlich mit erheblicher Hypertrophie der Gelenkschleimhaut und ausgeprägten Ergüssen im Kniegelenk als Folge eines massiven Abriebes der implantierten Prothesenteile, die z.T. aus Polyethylen bestehen und mit diesem Material dann auch ein ungünstiges Gleitverhalten ergeben können, wenn ein solcher Abrieb zu groß wird oder eine Lockerung der meistens mittels Zapfen und einer Verschraubung und oftmals mit einer Zementierung vorgenommenen Knochenverankerung der Komponententeile der Prothese auftritt. Bei der Feststellung solcher Komplikationen muß häufig die Implantation einer neuen Endoprothese veranlaßt werden, wobei sich neue Probleme bspw. hinsichtlich der Schaffung einer geänderten Auflage für die Komponenten einer neuen Prothese mit dem Erfordernis einer Resektion von weiteren Knochenteilen ergeben.

Der Erfindung liegt daher die **Aufgabe** zugrunde, ein Verfahren zur Herstellung einer Endoprothese als Gelenkersatz insbesondere bei Kniegelenken bereitzustellen, welches die bei der herkömmlichen Implantation solcher Endoprothesen feststellbaren Komplikationen minimieren hilft, wie bspw. die unphysiologische Gelenkbelastung, die Problematik einer ausreichenden Verankerungsmöglichkeit insbesondere der femoralen und tibialen Prothesenkomponenten und die Vermeidung eines zu großen Knochenverlustes primär bei einer erstmaligen Implantation einer solchen Endoprothese als Gelenkersatz bei Kniegelenken.

Diese Aufgabe wird bei einem Verfahren zur Herstellung einer Endoprothese als Gelenkersatz insbesondere bei Kniegelenken dadurch gelöst, daß von den folgenden Stufen Gebrauch gemacht wird:
1. Es wird ein präoperatives Bild von dem geschädigten Kniegelenk des Patienten hergestellt. Die Herstellung eines solchen Bildes kann dabei mittels der Computer-Tomographie, also einem Schichtaufnahmeverfahren, vorgenommen werden oder bevorzugt mittels der Kernspintomographie, weil damit eine besonders scharfe Abgrenzung der Gelenkkontur durch Darstellung des Knorpelgewebes bzw. anderer Weichteile des geschädigten Kniegelenks möglich sind und damit auch eine entsprechend optimale Voraussetzung für den operativen Eingriff geschaffen wird.
2. Im Anschluß an die Herstellung eines solchen präoperativen Bildes wird der operative Eingriff am Femur, der Tibia und der Patella des geschädigten Kniegelenks vorgenommen. Bei diesem operativen Eingriff hat grundsätzlich nur eine vollständige Entfernung des nicht tragfähigen Knochens zu erfolgen und zusätzlich die Entfernung nur eines absoluten Minimums des angrenzenden gesunden Knochens wenigstens am Femur und der Tibia, sodaß an dem Knochen eine Resektionsfläche erhalten wird, die sich ideal darstellt für ein späteres Zementieren der zugehörigen femoralen bzw. tibialen Komponente der zu implantierenden Endoprothese.
3. Wenn der operative Eingriff abgeschlossen ist, dann wird von dem Kniegelenk ein entsprechendes postoperatives Bild hergestellt, dabei wieder entweder mit der Computer-Tomographie oder bevorzugt mit der Kernspintomographie.
4. Im Anschluß an diesen operativen Eingriff oder auch bereits im Anschluß an die Herstellung des präoperativen Bildes von dem geschädigten Kniegelenk wird dann eine Korrektur dieses präoperativen Bildes vorgenommen, wobei mit dieser Korrektur eine Annäherung an die bei einem gesunden Kniegelenk vorliegenden Verhältnisse gesucht wird. Diese Korrektur des präoperativen Bildes kann entweder manuell an diesem präoperativen Bild selbst vorgenommen werden, wobei mit der Korrektur also die mehr oder weniger idealen Konturen wenigstens des Femur und der Tibia nachgefahren werden, die für die mit der später implantierten Endoprothese bereitgestellten Gelenkflächen eine entsprechend optimale physiologische Gelenkkontur des Kniegelenks ergeben. Diese Korrektur des präoperativen Bildes kann daher alternativ auch mit der Zugrundelegung eines Bildes bewirkt werden, welches soweit noch möglich eine Spiegelbildaufnahme eines zu dem geschädigten Kniegelenk gegenseitigen, gesunden Kniegelenks ist, wobei dazu die Annahme vorausgesetzt wird, daß die beiden Kniegelenke eines Patienten gleich ausgebildet sind und daher über einen solchen Vergleich eines geschädigten mit einem gesunden Kniegelenk für das geschädigte Gelenk tatsächlich die günstigsten Voraussetzungen für die Implantation der Endoprothese zugrunde gelegt werden können. Daneben ist es grundsätzlich auch denkbar, daß die Korrektur des präoperativen Bildes durch einen Vergleich mit Bildern von Kniegelenken vorgenommen wird, die unter vergleichbaren Verhältnissen aufgenommen wurden, wobei die Kniegelenke mit dem geschädigten Kniegelenk vergleichbare Gelenkflächen von Femur, Tibia und Patella aufweisen.
5. Das somit korrigierte Präoperative Bild wird dann mit dem im Anschluß an den operativen Eingriff hergestellten postoperativen Bild verglichen, um die Unterschiede zwischen den beiden Bildern zu ermitteln. Dabei interessieren insbesondere die Verhältnisse an den Konturen von Femur und Tibia, da der Größenunterschied dieser Konturen die Basis für die anschließende Herstellung von entsprechenden femoralen und tibialen Komponenten der Endoprothese ergeben.
6. Wie vorstehend angegeben, betrifft somit der letzte Verfahrensschritt der Herstellung einer Endoprothese als Gelenkersatz bei Kniegelenken die Herstellung wenigstens von femoralen und tibialen Komponenten, welche dem mit dem Vergleichsbild ermittelten Größenunterschied der Flächen entspricht, die bei dem Femur und der Tibia für die korrigierten präoperativen Verhältnisse aufgenommen wurden. Die Herstellung solcher femoraler und tibialer Komponenten einer dann anschließend zu implantierenden Endoprothese kann dabei bspw. damit erfolgen, daß das die Unterschiede zwischen dem korrigierten präoperativen Bild und dem postoperativen Bild erfassende Vergleichsbild digitalisiert und dann für eine maschinelle Herstellung der Komponenten in einem Kopierverfahren verwendet wird.

Als Ergebnis des Verfahrens zur Herstellung einer Endoprothese als Gelenkersatz bei Kniegelenken werden damit Komponenten erhalten, welche somit die Konturen des gesunden Kniegelenks aufweisen bzw. allenfalls geringfügig unterschiedliche, den aktuellen Knochen-Weichteilverhältnissen angepaßte, dabei physiologisch gleichzeitig entsprechend ideal angepaßte Gelenkkonturen ergeben, deren erfolgreiche Implantation dann nur noch mehr oder weniger von der Güte der Verankerung der Komponenten abhängig ist. Weil für die Implantation solcher nahezu idealer Gelenkersatzkomponenten eine größere mechanische Lockerungsgefahr kaum zu erwarten ist, bietet sich für ihre Verankerung ideal eine zementfreie Verankerung der Komponenten am Femur bzw. an der Tibia an, wobei im Hinblick auf die individuelle Anpassung der Gelenkverhältnisse bei einem Patienten der operative Eingriff an dem geschädigten Kniegelenk hinsichtlich der zusätzlichen Wegnahme von gesundem Knochen neben der vollständigen Entfernung von nicht mehr tragfähigem Knochen bereits so ausgerichtet werden kann, daß für die Implantation eine physiologisch einwandfreie Verankerung der Komponenten der Endoprothese erhalten wird.

Sofern erforderlich, wird die Herstellung einer Endoprothese selbstverständlich auch die Herstellung einer Komponente einschließen, die für die Patella des geschädigten Kniegelenks verwendet wird. Das Verfahren ist daneben auch anwendbar für einen operativen Eingriff bei anderen Gelenken, so bspw. auch am Sprunggelenk, wenn insoweit vergleichbare Verhältnisse voraussetzbar sind.

## Patentansprüche

1. Verfahren zur Herstellung einer Endoprothese als Gelenkersatz bei Kniegelenken, wobei die Prothese einen operativen Eingriff bei dem Femur, der Tibia und der Patella eines geschädigten Kniegelenks voraussetzt, bestehend aus den Stufen:
a) Herstellung eines präoperativen Bildes von dem geschädigten Kniegelenk;
b) Korrektur des präoperativen Bildes von dem geschädigten Kniegelenk hinsichtlich einer Annäherung an die bei einem gesunden Kniegelenk vorliegenden Konturen wenigstens am Femur und an der Tibia;
c) Herstellung eines postoperativen Bildes von dem geschädigten Kniegelenk;
d) Gegenüberstellung des korrigierten präoperativen Bildes und des postoperativen Bildes von dem geschädigten Kniegelenk auf einem Vergleichsbild für eine Ermittlung des Unterschiedes zwischen den beiden Bildern von dem geschädigten Kniegelenk; und
e) Herstellung wenigstens einer femoralen und einer tibialen Komponente einer Endoprothese auf der Basis der mit dem Vergleichsbild ermittelten Unterschiede der entsprechenden Konturen von Femur und Tibia bei dem korrigierten präoperativen Bild und dem postoperativen Bild von dem geschädigten Kniegelenk.

2. Verfahren nach Anspruch 1, bei welchem die Bilder von dem geschädigten Kniegelenk mittels der Computer-Tomographie oder der Kernspintomographie hergestellt werden.

3. Verfahren nach Anspruch 1 oder 2, bei welchem die Korrektur des Präoperativen Bildes manuelle vorgenommen wird.

4. Verfahren nach einem der Ansprüche 1 bis 3, bei welchem die Korrektur des präoperativen Bildes mit dem Bild einer Spiegelbildaufnahme eines zu dem geschädigten Kniegelenk gegenseitigen, gesunden Kniegelenks des Patienten vorgenommen wird.

5. Verfahren nach einem der Ansprüche 1 bis 4, bei welchem die Korrektur des präopertiven Bildes durch einen Vergleich mit Bildern von gesunden Kniegelenken vorgenommen wird, bei denen an den Gelenkflächen wenigstens des Femur und der Tibia mit dem präoperativen Bild des geschädigten Kniegelenks vergleichbare Konturen vorliegen.

6. Verfahren nach einem der Ansprüche 1 bis 5, bei welchem das Vergleichsbild digitalisiert und für die maschinelle Herstellung der femoralen und tibialen Komponenten der Endoprothese in einem Kopierverfahren verwendet wird.
